**Europäisches Patentamt**

**European Patent Office**

**Office européen des brevets**

(11) Veröffentlichungsnummer: **0 189 546**
**B1**

(12) **EUROPÄISCHE PATENTSCHRIFT**

(45) Veröffentlichungstag der Patentschrift:
**22.03.89**

(21) Anmeldenummer: **85114887.4**

(22) Anmeldetag: **23.11.85**

(51) Int. Cl.⁴: **A 61 F 2/30**

(54) Poröse metallische Auflage zur mindestens teilweisen Bedeckung von Implantatoberflächen.

(30) Priorität: **09.01.85 CH 82/85**

(43) Veröffentlichungstag der Anmeldung:
**06.08.86 Patentblatt 86/32**

(45) Bekanntmachung des Hinweises auf die Patenterteilung:
**22.03.89 Patentblatt 89/12**

(84) Benannte Vertragsstaaten:
**AT DE FR GB IT**

(56) Entgegenhaltungen:
**EP-A- 0 016 480**
**DE-A- 2 404 214**
**DE-A- 2 808 740**
**DE-A- 2 842 847**
**DE-A- 3 036 520**
**DE-A- 3 130 732**
**GB-A- 2 024 631**
**US-A- 3 906 550**
**US-A- 4 479 271**
**US-A- 4 492 577**

(73) Patentinhaber: **GEBRÜDER SULZER AKTIENGESELLSCHAFT, Zürcherstrasse 9, CH-8401 Winterthur (CH)**

(72) Erfinder: **Frey, Otto, Wallrütistrasse 56, CH-8400 Winterthur (CH)**
Erfinder: **Semlitsch, Manfred, Dr., Endlikerstrasse 86, CH-8400 Winterthur (CH)**
Erfinder: **Weber, Heinz, Bulligerstrasse 1, CH-8400 Winterthur (CH)**

(74) Vertreter: **Dipl.-Ing. H. Marsch Dipl.-Ing. K. Sparing Dipl.-Phys.Dr. W.H. Röhl Patentanwälte, Rethelstrasse 123, D-4000 Düsseldorf 1 (DE)**

# Beschreibung

Die Erfindung betrifft eine poröse metallische Auflage zur mindestens teilweisen Bedeckung von metallischen Implantatoberflächen, insbesondere von Verankerungselementen für Endoprothesen, bestehend aus mehreren Lagen übereinander geschichteter Drahtnetze, bei denen die Porengrösse der einzelnen Lagen von aussen zur Oberfläche des Implantats hin abnimmt.

Eine Oberflächenstruktur der vorstehend genannten Art ist aus der US-A-4 492 577 bekannt. Als bevorzugte Herstellungsart für die dortigen Implantate werden Giessverfahren genannt, bei denen der metallische Kern in ein zuvor aus geeignetem Draht gewobenes, in einer Giessform untergebrachtes Netz eingegossen wird. Weiterhin wird erwähnt, dass die poröse Struktur jedoch auch durch Schweissen, Sintern oder Kleben mit dem Kern verbunden werden kann

Bei orthopädischen Implantaten sind beispielsweise für Verankerungsschäfte die Implantatkerne häufig geschmiedet. Darüberhinaus besteht bei den geschilderten Giessverfahren die Gefahr, dass die feinen Drähte eines Strukturnetzes mindestens teilweise von dem flüssigen Kernmaterial verbrannt und zerstört werden. Die Herstellung von mit porösen Auflagen versehenen Implantatkernen nach dem vorstehend skizzierten Giessverfahren hat sich daher nicht bewährt.

Weiter hin treten bei Schweiss- und Sinterverfahren häufig thermische Belastungen des Implantatkernes auf, die zu unerwünschten Gefügeänderungen und dadurch zur Verschlechterung der mechanischen Eigenschaften, wie z.B. der Festigkeit oder der Zähigkeit, führen.

Es ist daher die Aufgabe der vorliegenden Erfindung, eine poröse metallische Auflage auf einem metallischen Implantatkern zu befestigen, ohne dass die vorstehend geschilderten Nachteile in Kauf genommen werden müssen. Diese Aufgabe wird dadurch gelöst, dass die Lagen der Drahtnetze auf einer Zwischenplatte aufgebracht sind, die auf der Oberfläche des Implantates befestigbar ist.

Die poröse Auflage wird bei der Erfindung durch Sintern (Diffusionsschweissung) mit der Zwischenplatte fest verbunden, bevor diese ihrerseits, beispielsweise durch einzelne Punktschweissungen, auf den Kern geheftet oder durch mechanische Verbindungen, wie Schrauben, mit dem Kern verbunden wird.

Aus der US-PS 4 479 271 ist ein Implantat bekannt, bei dem eine poröse Auflage über eine Zwischenplatte in einem Kunststoffteil befestigt wird. Bei der Herstellung wird der verflüssigte Kunststoff bis zu einer gewissen Tiefe in die poröse Auflage eingegossen und erstarrt. Da hierbei nur relativ niedrige Temperaturen erreicht werden, besteht die Gefahr von Gefüge- und Festigkeitsänderungen in einem massiven metallischen Kern eines Implantates in diesem Falle nicht.

Ein weiterer Vorteil der Zwischenplatte besteht darin, dass diese im allgemeinen aus einem gewebefreundlichen Material besteht, und anwachsendes Gewebe vom Kern des Implantates, der aus weniger gewebefreundlichen Werkstoffen hergestellt sein kann, isoliert. Darüberhinaus bildet sie eine definierte Grenze, bis zu der Gewebe einwachsen kann.

Um die Kontaktfläche für das Gewebe besonders bei Verwendung von Drahtnetzen zu vergrössern und die einzelnen Lagen zu glätten, ist es – wie an sich bekannt – zweckmässig, wenn die geschichteten Lagen der Metallgitter als Ganzes oder mindestens die Gitter einzelner Lagen vor der Schichtung gewalzt werden.

Aus dem Experiment heraus hat es sich für die porösen Strukturen als besonders geeignet erwiesen, wenn die lichten Weiten und/oder die Stegbreiten des Gitters absolut zwischen 0,05 und 1,5 mm und/oder wenn die Porenvolumen in den einzelnen Lagen 20–90% betragen.

Im folgenden wird die Erfindung anhand von Ausführungsbeispielen im Zusammenhang mit der Zeichnung näher erläutert.

Fig. 1 ist ein Querschnitt durch ein erstes Ausführungsbeispiel der neuen Auflage auf einer – nur in einem Ausschnitt vergrössert dargestellten – Implantatoberfläche;

Fig. 2 ist in gleicher Darstellung wie Fig. 1 eine zweite Ausführungsform der Erfindung.

Das Implantat, das beispielsweise ein metallener Verankerungsschaft 1 einer Hüftgelenksendoprothese ist, trägt eine Auflage 2; diese bildet eine gewebefreundliche Oberflächenstruktur, in die im Laufe der Zeit Knochengewebe einwächst, wodurch der Schaft 1 zementfrei im Knochen verankert wird

Die Auflage 2, die teilweise in einer Vertiefung 3 des Schaftes 1 liegt, besteht von innen nach aussen aus einer Zwischenplatte 4 und aus drei Lagen oder Schichten aus Drahtnetze 5. Durchmesser und Maschenweite der Drahtnetze nehmen von innen nach aussen zu, so dass sich ihre Porengrösse stetig, d.h. ohne sich in einer Zwischenlage zu verringern, vergrössert. So hat die innerste Lage 5a beispielsweise Drähte 6 mit einem Durchmesser von 0,1 mm und eine Maschenweite des Netzes von 0,2 mm; in der mittleren Lage 6b sind die entsprechenden Werte 0,25 mm und 0,5 mm, während sie bei der äussersten Lage 5c 0,5 mm und 1 mm betragen.

Die Auflage 2 besteht aus einem der als besonders gewebefreundlich bekannten Metalle, wie Titan, Tantal, Niob, Zirkon oder Legierungen mit diesen Metallen als Basiswerkstoffe. Bei der Herstellung wird die Auflage 2 zunächst losgelöst vom Implantat 1 zusammengefügt, wobei die Ausbildung einer metallurgischen Bindung zwischen den einzelnen Lagen 5a–5c und die Zwischenplatte 4 bevorzugt wird. Die einzelnen Lagen 5a–5c und die Zwischenplatte 4 werden dabei am einfachsten durch Sintern oder Schweissen untereinander verbunden. Es sind jedoch auch andere Verbindungen, wie z.B. Klebeverbindungen, möglich. In einem zweiten Schritt wird die Auflage 3 als Ganzes dann auf dem Implantat 1, vorzugsweise durch Punktschweissen, an einer Anzahl Haftstellen befestigt. Die thermischen Belastungen des Kernes durch lokale Punktschweissungen haben auf das Gefüge des Kernes nur einen begrenzten Einfluss, der zu keinem die mechanischen Eigenschaften beeinträchtigenden Gefügeänderungen führt.

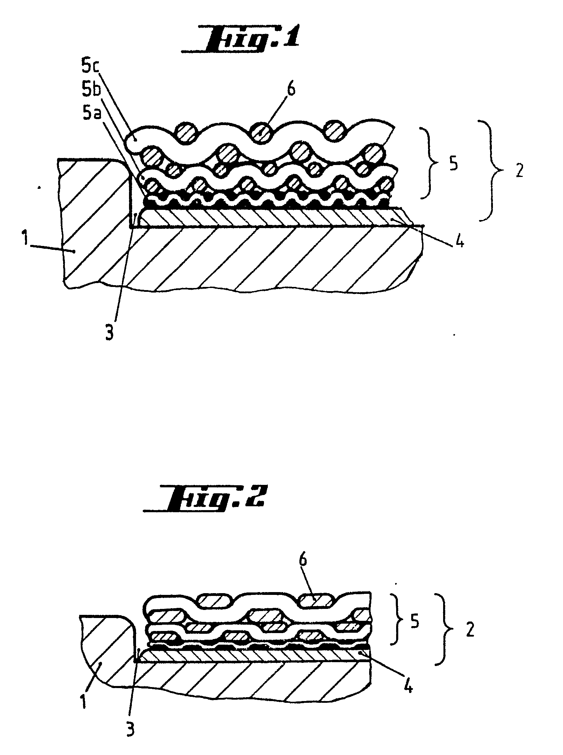

Bei der Ausführungsform nach Fig. 2 sind die ebenfalls aus Drähten 6 gewobenen einzelnen Lagen 5 vor dem Aufbau der Auflage 2 durch Walzen verformt worden, um bei gleichbleibenden Drahtdurchmessern einerseits die Oberfläche der Drähte 6, an die das Knochengewebe anwachsen soll, zu vergrössern und andererseits die «Dicke» der Auflage 2 zu reduzieren.

## Patentansprüche

1. Poröse metallische Auflage zu mindestens teilweisen Bedeckung von metallischen Implantatoberflächen, insbesondere von Verankerungselementen für Endoprothesen, bestehend aus mehreren Lagen übereinander geschichteter Drahtnetze (5a–5c), bei denen die Porengrösse der einzelnen Lagen von aussen zur Oberfläche des Implantates (1) hin abnimmt, dadurch gekennzeichnet, dass die Lagen (5a–5c) der Drahtnetze (5) auf einer Zwischenplatte (4) aufgebracht sind, die auf der Oberfläche des Implantates (1) befestigbar ist.

2. Auflage nach Anspruch 1, dadurch gekennzeichnet, dass die Drahtnetze (5) mindestens einzelner Lagen (5a–5c) vor ihrer Schichtung gewalzt sind.

3. Auflage nach einem der Ansprüche 1 und 2, dadurch gekennzeichnet, dass die lichten Weiten und die Stegbreiten der Gitter in den einzelnen Lagen (5a–5c) zwischen 50 µm und 1,5 mm betragen.

4. Auflage nach einem der Ansprüche 1–3, dadurch gekennzeichnet, dass das Porenvolumen in den einzelnen Lagen (5a–5c) 20–90% beträgt.

## Revendications

1. Structure de revêtement poreuse et métallique pour recouvrir au moins partiellement des surfaces métalliques d'implants, en particulier des éléments d'ancrage pour endoprothèses, constituée par plusieurs couches d'entrelacs de fils métalliques (5a–5c) stratifiés en superposition, dans lesquels la grosseur des pores des couches individuelles diminue de l'extérieur vers la surface de l'implant (1), caractérisée par le fait que les couches (5a–5c) des entrelacs (5) de fils métalliques sont placées sur une plaquette intercalaire (4) pouvant être fixée sur la surface de l'implant (1).

2. Structure de revêtement selon la revendication 1, caractérisée par le fait que les entrelacs (5) de fils métalliques d'au moins certaines couches individuelles (5a–5c) sont laminés préalablement à leur stratification.

3. Structure de revêtement selon l'une des revendication 1 et 2, caractérisée par le fait que les intervalles internes et les largeurs des membrures des tressages sont compris, dans les couches individuelles (5a–5c), entre 50 µm et 1,5 mm.

4. Structure de revêtement selon l'une des revendications 1–3, caractérisée par le fait que le volume des pores est de 20–90% dans les couches individuelles (5a–5c).

## Claims

1. A porous metal capping providing at least partial covering of metal implant, surfaces, more particularly of fixing elements for endoprotheses, the capping comprising a number of layers of wire meshes (5a–5c) which are disposed one above another, the pore size of the discrete layers decreasing from the outside towards the surface of the implant (1), characterised in that the layers (5a–5c) of the wire meshes (5) are disposed on an intermediate plate (4) securable to the implant surface.

2. A capping according to claim 1, characterised in that the wire meshes (5) of at least individual layers (5a–5c) are rolled before layering.

3. A capping according to claim 1 and/or 2, characterised in that the internal widths and the web widths of the meshes in the discrete layers (5a–5c) are between 50 µm and 1,5 mm.

4. A capping according to any of claims 1–3, characterised in that the pores occupy from 20 to 90% of the volume of the discrete layers (5a–5c).

0189546

**Fig. 1**

**Fig. 2**